(19) Europäisches Patentamt
European Patent Office
Office européen des brevets

(11) Publication number: 0 298 890 A2

(12) # EUROPEAN PATENT APPLICATION

(21) Application number: 88500069.5

(51) Int. Cl.4: C 07 C 93/20

(22) Date of filing: 01.07.88

(30) Priority: 06.07.87 ES 8701972

(43) Date of publication of application: 11.01.89 Bulletin 89/02

(84) Designated Contracting States: BE DE ES FR GB GR IT LU NL

(71) Applicant: Sune Coma, Nuria
Ganduxer 22, 5 piso 1a
E-08021 Barcelona (ES)

(72) Inventor: Sune Coma, Nuria
Ganduxer 22, 5 piso 1a
E-08021 Barcelona (ES)

(74) Representative: Curell Sunol, Jorge et al
c/o Dr. Ing. M. Curell Sunol I.I. S.L. Passeig de Gràcia 65 bis
E-08008 Barcelona (ES)

Claims for the following Contracting States: ES + GR.

(54) **New 2-(P-isobutylphenyl)-propionic acid ester and process for the preparation thereof.**

(57) The new ester is N,N-dimethylaminoethanol 2-(p-isobutylphenyl)-propionate.

The process comprises reacting 2-(p-isobutylphenyl)-propionic acid with thionyl chloride to provide the corresponding acid chloride, which is reacted with N,N-dimethylaminoethanol to give the new ester.

EP 0 298 890 A2

**Description**

**NEW 2-(p-ISOBUTYLPHENYL)-PROPIONIC ACID ESTER AND PROCESS FOR THE PREPARATION THEREOF.**

This invention relates to a new 2-(p-isobutylphenyl)-propionic acid ester of the structural formula I

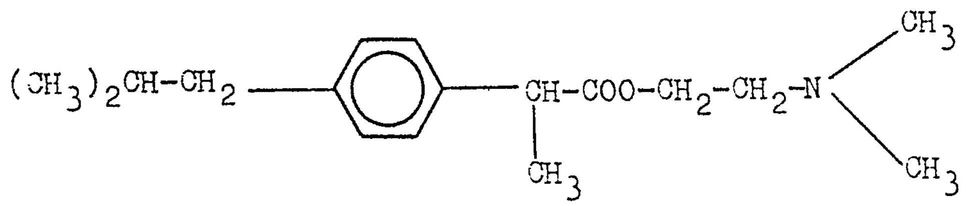

(I)

and to a process for the preparation thereof.

This compound is, obviously, a derivative of 2-(p-isobutylphenyl)-propionic acid of formula II

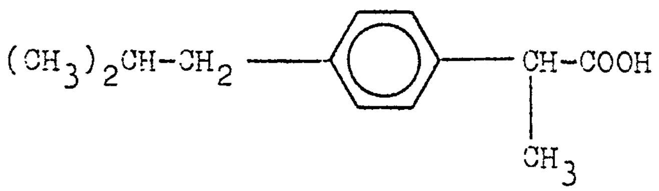

(II)

which has interesting pharmacological properties and is used as an analgesic and/or antiinflammatory drug. It has been discovered, surprisingly, that the formula I ester has more favourable pharmacological properties and is more soluble in water that the formula II acid.

The process is characterised in that the acid of formula II is reacted with thionyl chloride in a first inert solvent, such as chloroform, methylene chloride, anhydrous benzene, toluene and in the presence of a catalyst such as dimethylformamide, to give the acid chloride of formula III

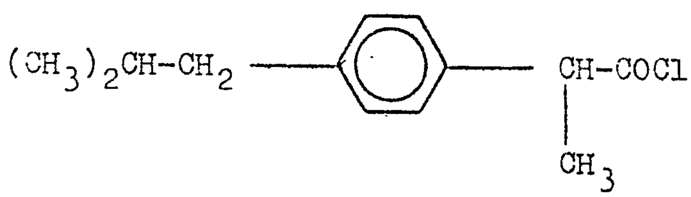

(III)

which is reacted with N,N-dimethylaminoethanol of formula IV

$(CH_3)_2N\text{-}CH_2\text{-}CH_2OH$ (IV)

in the presence of a second inert solvent (such as chloroform, dichloromethane, benzene, ethyl ether, terc. butylmethyl ether, acetone), to give the final compound of formula I.

The reaction temperature of both phases of the process may lie between 20° and 150° C. The reactions are normally carried out at the boiling point of the solvent used.

The reactions may be carried out at normal pressure, but also at high pressure. Generally atmospheric pressure is used.

According to the invention, the formula I compound is isolated and separated by techniques generally used for this purpose, it being possible to subject the product to a traditional purification such as recrystallisation or chromatography.

The formula I compound may be administered in the form of a pharmacologically acceptable salt. Such forms of salt have the same activity level and are easily prepared in known ways. This invention also provides a pharmaceutical composition containing the compound of formula I associated with a pharmaceutical diluent or vehicle. Such compositions may be offered, for example, in solution or tablet form.

An indicated daily dose is approximately 100 mg to 2,000 mg. suitably administered from 2 to 4 times daily in divided doses of from about 100 mg to about 500 mg, or in form of a delayed action preparation.

The following Example illustrates the invention, without limiting it in any way.

EXAMPLE:

A) Preparation of 2-(p-isobutylphenyl)-propionic acid chloride.

36 ml of thionyl chloride and 3 ml of dimethylformamide were added to a suspension of 51.6 g of 2-(p-isobutylphenyl)-propionic acid in 400 ml of chloroform. The mixture was heated under reflux for 90 minutes. After cooling the reaction mixture, the solvent was removed under vacuum. The thus obtained product is sufficiently pure to be used in the following reaction.

B) Preparation of N,N-dimethylaminoethanol 2-(p-isobutylphenyl)-propionate.

35.7 g of N.N-dimethylaminoethanol were added to a solution of 89.9 g of 2-(p-isobutylphenyl)-propionic acid chloride in 300 ml of methylene chloride. The reaction mixture was heated under reflux for 4 hours. At the end of this time, the mixture was cooled to room temperature and was washed three times with water. The solution was dried over anhydrous sodium sulphate and the solvent was then removed under vacuum. The residue was recrystallised from isopropanol. In this way, N,N-dimethylaminoethanol 2-(p-isobutylphenyl)-propionate hydrochloride was obtained, with an 80% yield and melting point of 124°-126°.
Elementary analysis for $C_{17}H_{28}ClNO_2$
Calculated: C: 65.05% H: 8.99% N: 4.46%
Found : C: 65.27% H: 9.04% N: 4.63%

PHARMACOLOGICAL ASSAY.

The compound of formula I prepared according to Example I has the following pharmacological properties compared with the acid of formula II:

| Compound | Antiinflammatory effect[(a)] ($ED_{50}$) | Analgesic effect[(b)] ($ED_{50}$) |
|---|---|---|
| I | $2.04 \times 10^{-4}$ | $0.86 \times 10^{-5}$ |
| II | $2.52 \times 10^{-4}$ | $2.91 \times 10^{-5}$ |

(a) carrageenin test p.o. in the rat. Value given in moles/kg.

(b) phenylbenzoquinone test p.o. in the rat. Value given in moles/kg.

These data show that the formula I compound has the same antiinflammatory activity and is twice as active as an analgesic as the formula II compound.

**Claims**

1.- An ester of 2-(p-isobutylphenyl)-propionic acid, having the chemical name of N,N-dimethylaminoethanol 2-(p-isobutylphenyl)-propionate of formula:

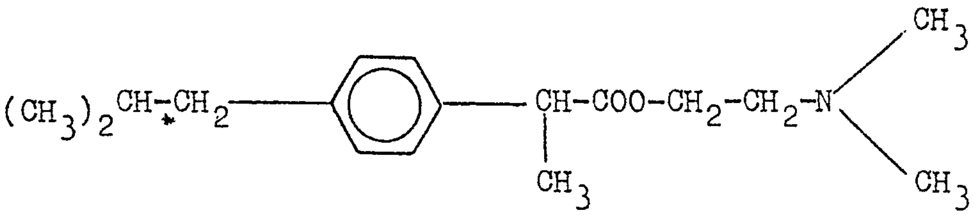

(I)

and the pharmaceutically acceptable salts thereof.

2.- A process for the preparation of an ester of 2-(p-isobutylphenyl)-propionic acid, having the structural formula I

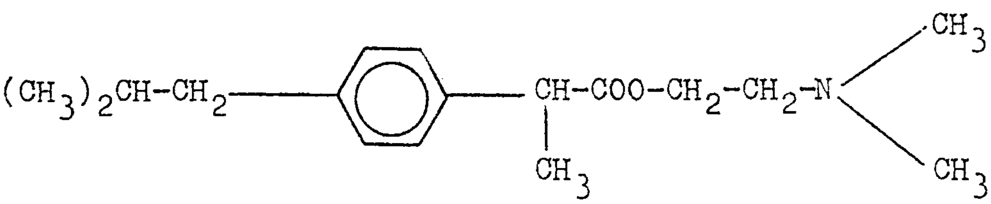

(I)

and salts thereof, characterised in that 2-(p-isobutylphenyl)-propionic acid of formula II

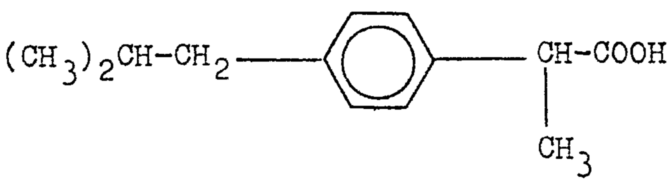

(II)

is reacted with thionyl chloride in the presence of a first inert solvent and a catalyst to give the acid chloride of formula III

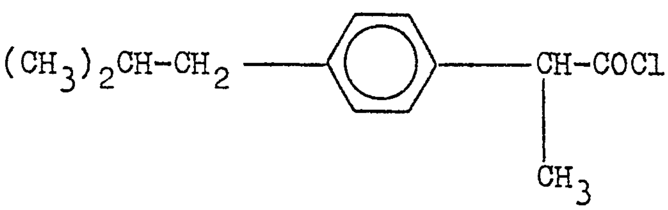

(III)

which is reacted with N,N-dimethylaminoethanol in the presence of a second inert solvent to give the compound of formula I.

3.- The process of claim 2, characterised in that said first inert solvent is chloroform.

4.- The process of claim 2 or 3, characterised in that said catalyst is dimethylformamide.

5.- The process of claim 2, 3 or 4, characterised in that said second inert solvent is methylene chloride.

**Claims for the following Contracting States : ES, GR**

1.- A process for the preparation of an ester of 2-(p-isobutylphenyl)-propionic acid, having the structural formula I

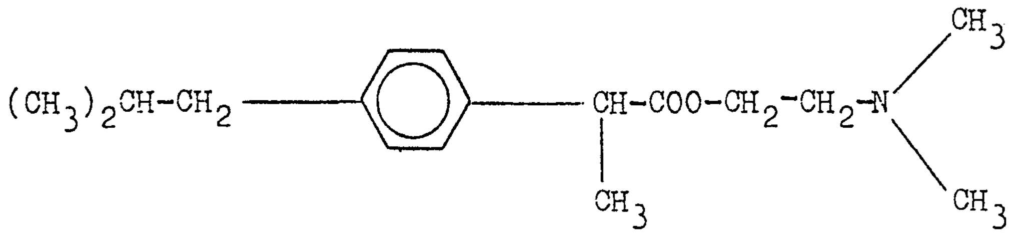

(I)

and salts thereof, characterised in that 2-(p-isobutylphenyl)-propionic acid of formula II

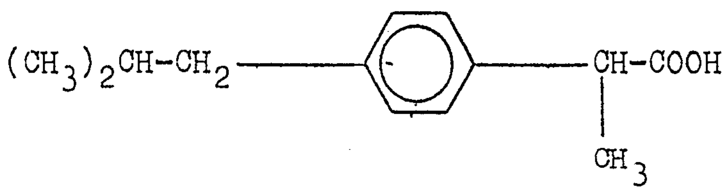

(II)

is reacted with thionyl chloride in the presence of a first inert solvent and a catalyst to give the acid chloride of formula III

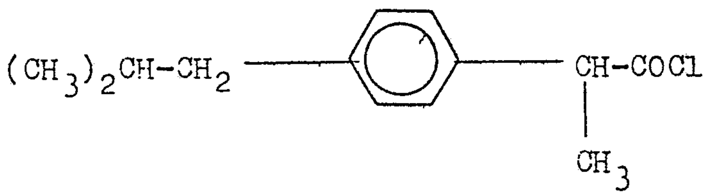

(III)

which is reacted with N,N-dimethylaminoethanol in the presence of a second inert solvent to give the compound of formula I.

2.- The process of claim 1, characterised in that said first inert solvent is chloroform.

3.- The process of claim 1 or 2, characterised in that said catalyst is dimethylformamide.

4.- The process of claim 1, 2 or 3, characterised in that said second inert solvent is methylene chloride.